# EUROPEAN PATENT SPECIFICATION

(11) **EP 1 496 948 B1**
(45) Date of publication and mention of the grant of the patent: **21.09.2005**
(21) Application number: 03711469.1
(22) Date of filing: 07.03.2003
(51) Int. Cl.: A61L 2/08, A62D 3/00, A61L 2/24

(54) **METHOD AND APPARATUS FOR DESTROYING MICROBIAL CONTAMINATION OF MAIL AND PAPER CURRENCY**
METHODE UND VORRICHTUNG ZUR DEKONTAMINATION VON POSTGUT UND PAPIERGELD
PROCEDE ET APPAREIL DE DESTRUCTION DE LA CONTAMINATION MICROBIENNE DU COURRIER ET DES BILLETS DE BANQUE

(30) Priority: 12.03.2002 US 95869
(43) Date of publication of application: 19.01.2005
(73) Proprietor: STERIS INC., California 92590 (US)
(72) Inventor: KORENEV, Sergey, A., Mundelein, IL 60060 (US); KORENEV, Anton, S., Springfield, NJ 07081 (US)
(74) Representative: Gasquet, Denis
(86) International application number: PCT/US2003/007050
(87) International publication number: WO 2003/077957

(56) References cited:
- FR-A- 2 773 715
- US-A- 3 779 706
- US-A- 3 780 308
- US-A- 4 305 000
- US-A- 5 496 302
- DATABASE WPI Section Ch, Week 199710 Derwent Publications Ltd., London, GB; Class D22, AN 1997-100607 XP002246843 & CN 1 078 402 A (LIN G), 17 November 1993 (1993-11-17)
- PATENT ABSTRACTS OF JAPAN vol. 2000, no. 09, 13 October 2000 (2000-10-13) & JP 2000 163629 A (HITACHI ASAHI ELECTRONICS CO LTD), 16 June 2000 (2000-06-16)

## Description

The present application relates to the destruction of microbes on relatively thin paper or plastic carriers. It finds particular application in conjunction with killing Anthrax spores and other biological contaminants in mail and will be described with particular reference thereto. It will also find application in the treatment of other thin items such as bank notes, tickets, checks, receipts, and the like.

Biological terrorism through the mails has become a serious problem. Recent events have shown the ease with which bioterrorists can deliver Anthrax and other biological weapons to targets using the postal system.

There are numerous known decontaminating systems. These include radiation, x-rays, e⁻ beams, and ultraviolet light, plasma methods on the basis of etching with glow discharge, gas decontamination, and ozone decontamination.

High energy electron beams are ill-suited for destroying microbes in the mail. High-energy electrons, with a kinetic energy in the range of 5-10 MeV use large electron accelerators and need relatively large radiation shields for safety. Moreover, these systems are very expensive and need specialized staff to operate them. Further, the electron beam delivers a very high Joule energy over a limited penetration range. This energy leads to heating not only of the microbes, but also of the surrounding envelopes. This can lead to overheating of the envelopes and other paper in the mail, possibly causing combustion. This heating can also soften plastic materials in the mail leading to its deformation and thermal bonding with surrounding materials. Heat can also destroy security characteristics of bank notes.

FR 2773715 discloses sterilization of exterior surfaces of packaging with a pulsed beam of electrons of energy 100-500 keV. US 5,496,302 discloses sterilizing containers for medical liquids with a pulsed electron beam.

Gamma radiation, due to its greater penetrating power, is more amenable to bulk decontamination of mail. However, this same increased penetrating power increases the necessary shielding. Typically, very thick containment rooms are needed. When radioisotopes are used as the radiation source, heavy water containment tanks are needed and depleted radioactive waste issues arise. X-rays generated through x-ray vacuum tubes are highly energy consumptive, generate large amounts of waste heat, and have similar shielding problems to gamma radiation.

Plasma radiation methods are effective for killing microbes in the air and on surfaces. However, plasma discharge is not amenable to killing microbes inside of envelopes.

ozone and other toxic gas methods are effective for killing microbes. However, obtaining penetration into envelopes or other paper products is slow. For toxic gases, further time is needed to evacuate the toxic gas back out of the interior of the envelopes at the end of the process. Also, strong oxidants, such as ozone, may alter some inks and dyes in any printing on or in the envelopes.

The present application provides a new and improved irradiation system that overcomes the above-referenced problems and others.

### Summary of the Invention

In accordance with one aspect of the present invention, an apparatus is provided for microbially decontaminating flat objects including bank notes. A cash storage box stores the bank notes. A dispenser or receiver means dispenses or receives bank notes. A conveyor means moves the flat objects individually through a treatment chamber between the cash storage box and the dispenser or receiver means. A radiation means generates radiation while each flat object is in the treatment chamber. The radiation means includes an election beam source of up to 500 keV which generates pulses of electrons. A sensor means senses each flat object entering the chamber. A trigger circuit means triggers the radiation means to irradiate each flat object as it passes through the treatment chamber.

In accordance with another aspect of the present invention, a method of decontaminating flat objects, including bank notes potentially containing microbial contamination is provided. Individual flat objects pass along a conveying path between a cash box and a bank note port through a treatment region. Radiation is generated including electron beam pulses of up to 500 keV. Each flat object entering the treatment region is sensed and irradiation of each flat object with a burst of radiation is triggered as it passes through the treatment region.

One advantage of the present application resides in its capability to deactivate microbial contamination such as, but not limited to, bacteria, viruses and spores on paper currency without damaging the currency itself or the security aspects of the paper currency.

One advantage of the present invention resides in its efficiency of destruction of spores (using radiation and thermal sterilization).

One advantage of the present invention resides in its safety relative to security signs for paper currency and other security documents.

One advantage of the present invention resides in its speed.

Another advantage of the present invention resides in its low shielding requirements.

Another advantage of the present invention resides in its high operator safety.

Another advantage of the present invention resides in its modest cost and simplicity of operation.

Still further advantages of the present invention will become apparent to those of ordinary skill in the art upon reading and understanding the following detailed description of the preferred embodiments.

### Brief Description of the Drawings

The invention may take form in various components and arrangements of components, and in various steps and arrangements of steps. The drawings are only for purposes of illustrating a preferred embodiment and are not to be construed as limiting the invention.
FIGURE 1a is a diagrammatic illustration of a mail or other flat object decontaminating system;
FIGURE 1b is illustrative of the irradiation of spores in an envelope;
FIGURE 2a is a diagrammatic illustration of a more detailed system for irradiating mail or other flat objects in accordance with the present invention;
FIGURE 2b is an alternate embodiment of the system of FIGURE 2a; and,
FIGURE 2c is another alternate embodiment of the system of FIGURE 2a.

### Detailed Description of the Preferred Embodiments

Broadly stated, an apparatus and method are provided that capable of destroying microbial contamination on both mail and paper currency. As used herein, microbial contamination shall refer to bacteria, viruses, spores, pathogenic biological material and other biological material capable of deactivation by radiative means.

### Deactivation of Microbial Contamination on Mail

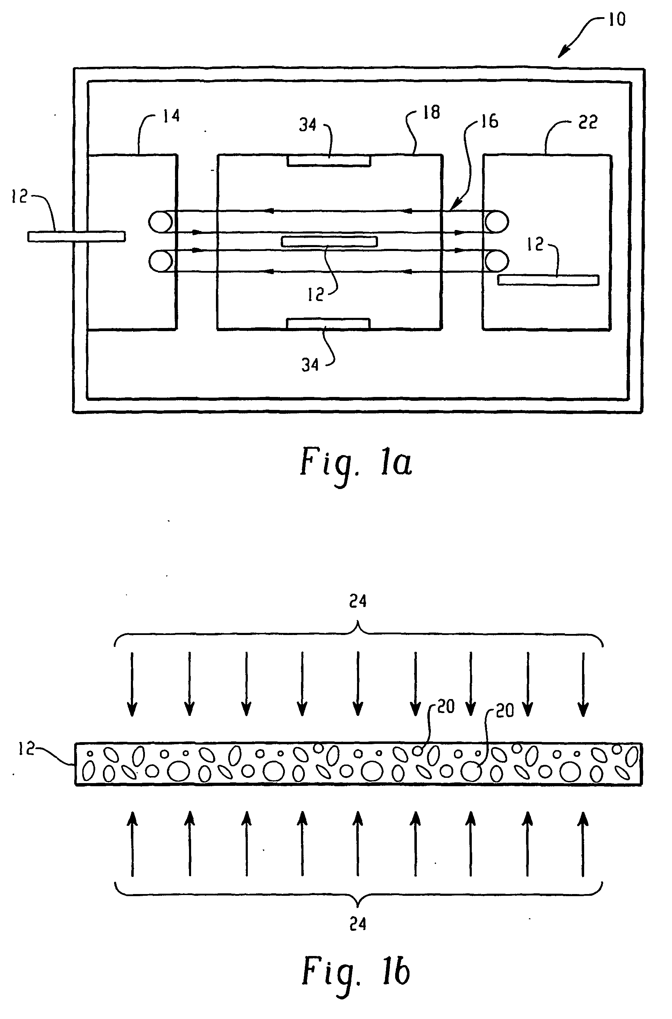

With reference to FIGURE 1a, an apparatus **10** receives a rapid supply of flat objects **12,** such as individual envelopes, letters, or mail traveling longitudinally at high speed substantially end-to-end. As an envelope enters a port **14,** it is identified and the power supply is actuated such that when a conveyor **16** conveys the envelope **12** into a treatment chamber **18,** it is irradiated under near adiabatic conditions. Energy from the radiation is absorbed by ionization and collision with atoms and molecules of microbial contamination **20** on and in the envelope leading to its deactivation. It is believed in one instance that in the case of microbes, the impact energy of the beam damages the microbe's DNA on a genetic level. It is further believed that thermal energy produced within the microbes resulting from the irradiation of the microbes deactivates the microbes. After being treated, the envelopes move to further processing stations or a storage box **22.**

As illustrated in FIGURE 1b, the envelopes **12** hold the Anthrax spores and other microbial contamination **20** against movement. In other paper products, microbes are analogously held in pores of the paper. It is believed in one instance that a pulsed energy beam **24** alters the genetic codes of the stationary microbes, thus deactivating the microbes, without destroying the envelope and other surrounding materials. In another instance, it is believed that thermal energy absorbed by the microbes as a result of the irradiation deactivates the microbes.

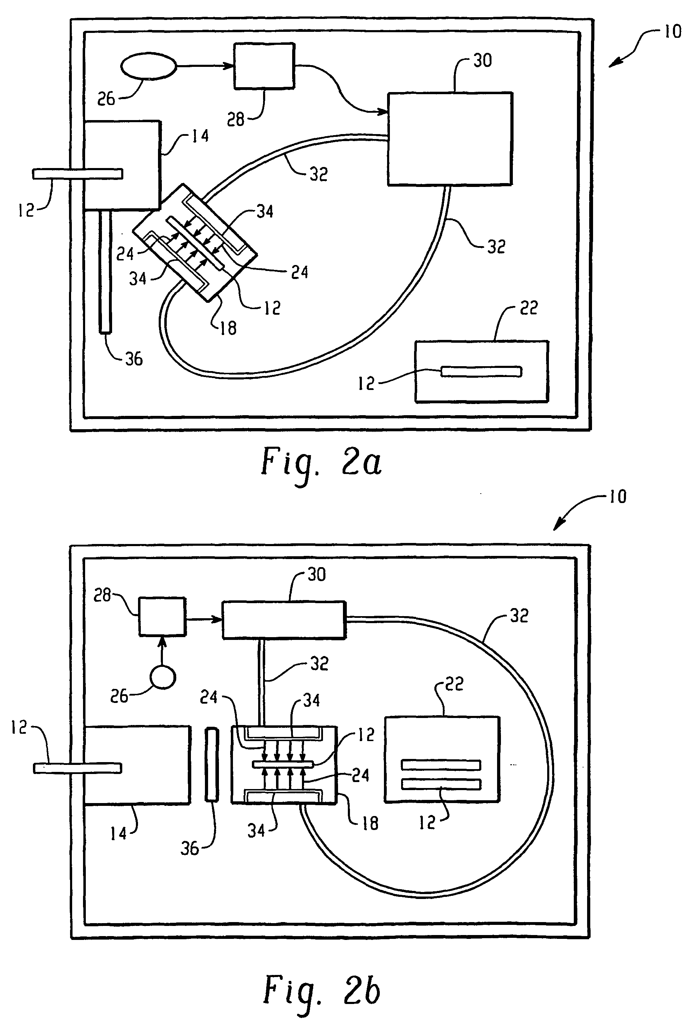

With reference to FIGURE 2a, when an envelope **12** enters the input port **14,** a sensor **26** sends a signal to a trigger signal generator **28.** The trigger signal generator triggers a pulse of a pulsed high voltage generator **30** to output one or more voltage pulses. The pulsed high voltage generator or power supply supplies pulses of energy by cables **32** to an electron or e⁻ beam generator **34.** The e⁻ beam generator is a low energy e⁻ beam generator, i.e., 500 keV or less that generates e⁻ beam pulses on the order of nanoseconds to microseconds in duration. In the preferred mail treatment embodiment, the e⁻ beam generator is a 500 keV generator that irradiates each sensed envelope for 100-500 nanoseconds. This produces about a 60 kilogray (kGy) radiation dose for one pulse. A 50 kGy dose of radiation is currently recommended to deactivate Anthrax spores. Of course, the pulse duration and its energy will be adjusted to accommodate the type of microbes or microbial contamination to be deactivated. In other embodiments, for less radiation resistant microbial contamination, an electron beam with an energy of only a few hundred electron volts may suffice to deactivate the microbial contamination. A 10 nanosecond pulse at 100 keV produces a 3 kGy dose which is sufficient to deactivate many common bacteria. *Strophylococcus aureus,* for example, is deactivated with a 0.65-2.6 kGy radiation dose. *Bacillus sublilis* is deactivated with a 2.6-12.9 kGy dose.

Preferably, a pair of the low energy e⁻ beam generators **34** is disposed on opposite sides of the envelope. At 200 keV, the e⁻ beams have very limited penetrating power, but are sufficient to penetrate normal thickness envelopes. However, because the penetration power is comparable with the thickness of conventional envelopes, relatively minor amounts of radiation shielding **36** are sufficient to prevent stray electrons from reaching the environment.

The timing and speed of the mail handling equipment that conveys the mail from the inlet port **14** to the treatment chamber **18** is coordinated with the speed of the trigger pulse generator **28** and the high voltage generator **30** such that the electron beam is formed when the envelope enters and as it passes through the chamber. After the envelope has been irradiated, it is passed to further mail handling and sorting equipment or stored in the buffer storage area **22**.

The throughput of envelopes on the mail conveyor is determined by the repetition rate of a pulsed, high voltage generator used to produce the electron beam. When the cross-section of the electron beam is comparable in size to the envelopes, an envelope process rate on the order of the repetition rate of the high voltage generator, used to generate the electron beam, is preferred. As is known in the industry, about 25% to 60% of the electron beam actually treats the microbial contamination in and/or on the mail. With a 500 kV high voltage generator, the maximum repetition rate of the generator is typically about 1 kHz. Thus, it is possible to treat about 1,000 envelopes/second with the method of the present application.

With reference to FIGURE 2b, the envelope **12** passes more directly from the input port **14** to the treatment chamber **18.** This facilitates higher mail handling speeds. A radiation shield **36** is placed between the input and the treatment chamber to prevent scattered and stray electrons from escaping at the input port **14.** In one embodiment, the shield **36** is in the form of a shutter that moves into position between the passage of each piece of mail. Alternately, the shield **36** is in the form of a stationary plate with a thin slot commensurate in size with the port **14.** The shield **36** can have an enlarged thickness adjacent the slot to prevent radiation at an acute angle from exiting. Once in the treatment chamber **18,** the mail can pause during treatment or can be treated during continuous movement. The presence of incoming mail is again sensed by a sensor **26** causing a trigger pulse generator **28** to generate a trigger pulse that causes a high voltage generator **30** to supply a high voltage pulse to the e⁻ beam generators **34.** The timing of the production of the e⁻ beams relative to the mail handling system is selected such one or more 20 nanosecond e⁻ beam pulses are generated during the time that the envelope is between the e⁻ beam generators. Preferably, the e⁻ beam is relatively wide enough to encompass the entire envelope in one shot. Alternately, the e⁻ beam is smaller than the envelope; but, the envelope is conveyed through the conveyor system such that the entire envelope is exposed to the e⁻ beam over two or more pulses. In this manner, deactivation of the microbial contamination contained within the envelope is effected.

Preferably, the treatment chamber **18** is built around a portion of the conveying system of in-place mail handling equipment. Alternately, a short extension or an additional section of the same mail conveyor system is placed in a mail sorting machine currently in use to move the mail through the treatment chamber **18.**

In the treatment of Anthrax spores, it is believed that electrons having a kinetic energy ranging from about 100 KeV to about 1,000 KeV (or equivalently, 1 MeV) are sufficient to deactivate the Anthrax spores. A combination of energetic electrons and x-rays can also be used to decontaminate mail housing Anthrax spores. In this regard, as known to those skilled in the art, x-rays can be produced from the electron beam. Both the electron beam and the x-rays can then be directed toward a source of Anthrax spores or other microbial contamination for deactivation.

It is also believed that the Anthrax spores are deactivated by the coupling of electron-irradiation damage done to the DNA of Anthrax spores and the irradiation induced thermal heating of the Anthrax spores to at least about 200° C.

### Deactivation of Microbial Contamination on Paper Currency

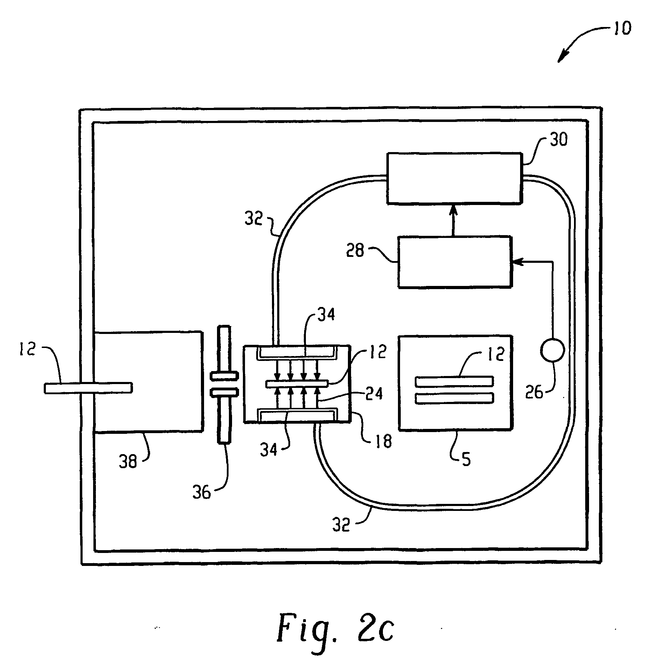

In an automatic teller machine embodiment (see FIGURE 2c), an ATM **10** receives flat objects **12** such as bills, bank notes, currency, or tickets at an input output dispenser **38.** The bills move at a slower speed through an irradiation chamber **18** into a cash storage box **22.** In ATMs, vending machines or ticket dispensers, the treatment chamber **18** is again built around a portion of the conveying or handling system.

The banknotes, tickets, or the like **12** are stored in the storage box or hopper **22.** As a bank note is fed out of the storage hopper, a sensor **26** causes a trigger circuit **28** to cause a power supply **30** to actuate e- beam generators **34.** Once the bill is irradiated, it is dispensed to the customer through the dispenser **38.**

In the case of decontaminating paper currency, electrons having an energy ranging from about 0.2 to about 25 KeV are believed to be sufficient to deactivate contamination on the surfaces thereof. One can also use a combination of electrons and soft x-rays, the soft x-rays produced from a cathode plasma, i.e., a plasma produced in the neighborhood of a cathode of a high voltage generator used to produce the beam of electrons, to decontaminate paper currency; however, electrons are the preferred vehicle for such decontamination. As is well known to those skilled in the art, x-rays can be produced from an electron beam. Both forms of radiation, i.e., electrons and x-rays, can then be used to deactivate microbial contamination on paper currency.

As mail, banks notes or paper currencies are irradiated, they absorb energy. In the case of e⁻ beam irradiation, Joule heat is dissipated to the depth of the e⁻ beam penetration. In the case of banknotes and mail, such heating may have deleterious effects. For example, such heating effects could adversely affect the security mechanism of at least some banknotes. In the case of mail, such heating effects could also soften adhesives used to seal envelopes, darken thermal printed labels, or simply destroy the paper of the envelopes. To overcome these deleterious effects, pulsed electron beams are used to irradiate flat objects. It is believed that the difference between the length in time duration of the pulses of e⁻ beams used (i.e., nanoseconds) and the length of times typically associated with the aforementioned chemical processes of degradation (i.e., microseconds to milliseconds) results in a nearly adiabatic treatment of the flat objects. In other words, the chemical processes involved to bring about the aforementioned degradations take anywhere from about 1,000 to about 1,000,000 times longer to play out than the time of exposure to one pulse of deactivating electrons.

The use of a pulsed electron beam also provides for a high dose of radiation without the need of a high power accelerator. By using a pulsed system, dosage levels ranging anywhere from about 30 to about 60 kGy can be attained without significant heating of the flat objects irradiated.

Although described in terms of e⁻ beam generators, it is to be appreciated that the same system may be used for x-ray or gamma-ray decontamination. The high energy e⁻ beam pulses can be applied to one or more of a bank of high energy x-ray tubes to effect an emission of x-ray pulses.

## Claims

1. An apparatus for microbially decontaminating flat objects **(12)** including bank notes, comprising:
a conveyor means **(16)** for moving the flat objects individually through a treatment chamber **(18);**
a radiation means **(34)** for generating radiation while each flat object is in the treatment chamber, the radiation means including a low energy electron beam source which generates pulses of electrons of up to 500 keV;
a sensor means **(26)** for sensing each flat object entering the treatment chamber;
a trigger means **(28)** for triggering the radiation means **(34)** to irradiate each flat object as it passes through the treatment chamber;
a cash storage box **(22)** in which bank notes are stored; and
a dispensing or receiving means **(38)** for at least one of dispensing and receiving the bank notes, the conveyor means conveying bank notes between the cash storage box and the dispensing or receiving means through the treatment chamber **(18) .**

2. The apparatus as set forth in claim 1 wherein the radiation means includes:
first and second radiation sources **(34)** disposed on opposite sides of the conveyor system.

3. The apparatus as set forth in either one of claims 1 and 2 wherein the electron beam source **(34)** includes means for generating pulses of electrons having a kinetic energy of 100-500 keV, the pulses lasting 20-1000 nanoseconds.

4. The apparatus as set forth in claim 3 wherein the means for generating the pulses generates electrons with a kinetic energy of 200 keV and the pulses last 20 nanoseconds.

5. The apparatus as set forth in claim 3 wherein the kinetic energy is 500 keV and the pulses last 100-500 nanoseconds.

6. The apparatus as set forth in any one of claims 1-5 wherein the conveyor means conveys bank notes deposited at the dispensing or receiving means **(38)** through the treatment chamber to the cash storage box **(22).**

7. The apparatus as set forth in any one of claims 1-6, wherein the radiation means **(34)** includes means for producing an electron beam having a cross-section comparable in size to an area of the irradiated flat object **(12).**

8. A method of decontaminating thin, flat objects **(12)** including bank notes potentially containing microbial contamination **(20),** the method comprising:
passing individual flat objects along a conveying path through a treatment region;
sensing each flat object entering the treatment region;
generating radiation including electron beam pulses of up to 500 keV;
triggering irradiating each flat object with a burst of the radiation, as it passes through the treatment region;
conveying the bank notes through the treatment region **(18)** between a cash box **(22)** and a bank note port **(14);** and
triggering the irradiating step as each bank note moves into the treatment region.

9. The method as set forth in claim 8 wherein the radiation includes electrons with an energy below 100 keV.

10. The method as set forth in either one of claims 8 and 9 wherein the radiation further includes x-rays.

11. The method as set forth in any one of claims 8-10 wherein the irradiating step includes:
irradiating the flat objects concurrently from opposite sides.

12. The method as set forth in any one of claims 8-11 wherein the irradiation step includes:
pulsing an electron beam to generate pulses of 20-1000 nanoseconds.

13. The method as set forth in any one of claims 8-12 wherein the irradiating step further includes:
generating electron beam pulses of electrons of 100-500 keV.

14. The method as set forth in any one of claims 8-13 further including:
receiving deposited bank notes at the bank note port;
conveying the deposited bank notes through the treatment region to the cash box;
triggering the irradiating as each deposited bank note moves through the treatment region.

15. The method as set forth in any one of claims 8-14, further including the step of heating the microbial contamination under nearly adiabatic conditions, the heat created by the irradiation step.

## Patentansprüche

1. Vorrichtung zur mikrobiellen Dekontamination flacher Gegenstände **(12)** einschließlich Banknoten, umfassend:
eine Transporteinrichtung **(16),** mit der die flachen Gegenstände einzeln durch eine Behandlungskammer **(18)** geführt werden;
eine Bestrahlungseinrichtung **(34)** zur Erzeugung einer Strahlung während des Verweilens des jeweiligen Gegenstands in der Behandlungskammer, wobei die Bestrahlungseinrichtung eine Elektronenstrahlquelle umfasst, die gepulste Elektronenstrahlen mit schwachen Energien von bis to 500 keV erzeugt;
einen Sensor **(26),** der jeden in die Behandlungskammer einlaufenden flachen Gegenstand erfasst;
eine Ansteuerung **(28),** die die Bestrahlungseinrichtung **(34)** ansteuert, so dass diese den jeweiligen Gegenstand beim Durchlaufen der Behandlungskammer bestrahlt;
einem Geldspeicherbehälter **(22)** zur Aufnahme von Banknoten; und
eine Ausgabe- oder Aufnahmeeinrichtung **(38),** die die Banknoten ausgibt und/oder aufnimmt, wobei die Transporteinrichtung die Banknoten auf dem Weg zwischen dem Geldspeicherbehälter und der Ausgabe- oder Aufnahmeeinrichtung durch die Behandlungskammer **(18)** befördert.

2. Vorrichtung nach Anspruch 1, **dadurch gekennzeichnet, dass** die Bestrahlungseinrichtung erste und zweite Bestrahlungsquellen **(34)** umfasst, welche auf einander gegenüberliegenden Seiten des Transportsystems angeordnet sind.

3. Vorrichtung nach einem der Ansprüche 1 und 2, **dadurch gekennzeichnet, dass** die Elektronenstrahlquelle **(34)** Mittel zur Erzeugung von Elektronenpulsen umfasst, die eine kinetische Energie von 100-500 keV aufweisen, wobei die Pulse eine Dauer von 20-1000 Nanosekunden haben.

4. Vorrichtung nach Anspruch 3, **dadurch gekennzeichnet, dass** die Mittel zur Erzeugung der Pulse Elektronen mit einer kinetischen Energie von 200 keV erzeugen, und die Pulse eine Dauer von 20 Nanosekunden haben.

5. Vorrichtung nach Anspruch 3, **dadurch gekennzeichnet, dass** die kinetische Energie 500 keV beträgt, und die Pulse eine Dauer von 100-500 Nanosekunden haben.

6. Vorrichtung nach einem der Ansprüche 1-5, **dadurch gekennzeichnet, dass** die Transporteinrichtung in der Ausgabe- oder Aufnahmeeinrichtung **(38)** abgelegte Banknoten durch die Behandlungskammer hindurch zum Geldspeicherbehälter **(22)** befördert.

7. Vorrichtung nach einem der Ansprüche 1-6, **dadurch gekennzeichnet, dass** die Bestrahlungseinrichtung **(34)** Mittel zur Erzeugung eines Elektronenstrahls umfasst, wobei der Elektronenstrahl einen Querschnitt aufweist, der in der Größe mit einem Bereich des zu bestrahlenden flachen Gegenstands **(12)** vergleichbar ist.

8. Verfahren zur Dekontaminierung möglicherweise mikrobiell kontaminierter **(20)** dünner, flacher Gegenstände **(12),** einschließlich Banknoten, umfassend folgende Verfahrensschritte:
Führen einzelner flacher Gegenstände entlang einer Transportbahn durch einen Behandlungsbereich;
Erfassen der jeweiligen in den Behandlungsbereich einlaufenden Gegenstände;
Erzeugen einer Strahlung umfassend gepulste Elektronenstrahlen von bis zu 500 keV;
Ansteuern des Bestrahlungsvorgangs, bei dem der jeweilige flache Gegenstand beim Durchlaufen des Behandlungsbereichs mit einem Strahlenbündel bestrahlt wird;
Befördern der Banknoten durch den Behandlungsbereich **(18)** auf dem Weg zwischen einem Geldbehälter **(22)** und einer Banknotenöffnung **(14);** und
Ansteuern des Bestrahlungsschrittes beim Einlaufen der jeweiligen Banknote in den Behandlungsbereich.

9. Verfahren nach Anspruch 8, **dadurch gekennzeichnet, dass** die Strahlung Elektronen mit einer Energie unterhalb von 100 keV umfasst.

10. Verfahren nach einem der Ansprüche 8 und 9, **dadurch gekennzeichnet, dass** die Strahlung ferner Röntgenstrahlen umfasst.

11. Verfahren nach einem der Ansprüche 8-10, **dadurch gekennzeichnet, dass** der Bestrahlungsschritt das gleichzeitige Bestrahlen der flachen Gegenstände von einander gegenüberliegenden Seiten umfasst.

12. Verfahren nach einem der Ansprüche 8-11, **dadurch gekennzeichnet, dass** der Bestrahlungsschritt das Pulsen eines Elektronenstrahls umfasst, so dass Elektronenpulse von 20-1000 Nanosekunden erzeugt werden.

13. Verfahren nach einem der Ansprüche 8-12, **dadurch gekennzeichnet, dass** der Bestrahlungsschritt ferner das Erzeugen von gepulsten Elektronenstrahlen umfasst, wobei die Elektronen eine Energie von 100-500 keV aufweisen.

14. Verfahren nach einem der Ansprüche 8-13, **dadurch gekennzeichnet, dass** es ferner folgende Verfahrensschritte umfasst:
Aufnehmen von abgelegten Banknoten an der Banknotenöffnung;
Befördern der abgelegten Banknoten durch den Behandlungsbereich bis zum Geldbehälter;
Ansteuern der Bestrahlung beim Durchlaufen der jeweiligen abgelegten Banknote durch den Behandlungsbereich.

15. Verfahren nach einem der Ansprüche 8-14, **dadurch gekennzeichnet, dass** es ferner den Verfahrenschritt umfasst, der darin besteht, die mikrobielle Kontamination unter annähernd adiabatischen Bedingungen zu erwärmen, wobei die Wärme durch den Bestrahlungsschritt entsteht.

## Revendications

1. Appareil pour une décontamination microbienne d'objets plats (12) comprenant des billets de banque, comprenant :
un moyen de transport (16) pour déplacer les objets plats individuellement à travers une chambre de traitement (18) ;
un moyen de rayonnement (34) pour générer un rayonnement tandis que chaque objet plat se trouve dans la chambre de traitement, le moyen de rayonnement comprenant une source de faisceau électronique à faible énergie qui génère des impulsions d'électrons allant jusqu'à 500 keV ;
un moyen de détection (26) pour détecter chaque objet plat entrant dans la chambre de traitement ;
un moyen de déclenchement (28) pour déclencher le moyen de rayonnement (34) pour exposer au rayonnement chaque objet plat au fur et à mesure qu'il passe à travers la chambre de traitement ;
une boîte de rangement pour des espèces (22) dans laquelle des billets de banque sont stockés ; et
un moyen de distribution ou de réception (38) pour au moins une parmi la distribution et la réception des billets de banque, le moyen de transport transportant des billets de banque entre la boîte de rangement pour des espèces et le moyen de distribution ou de réception à travers la chambre de traitement (18).

2. Appareil selon la revendication 1, dans lequel le moyen de rayonnement comprend :
des première et seconde sources de rayonnement (34) disposées sur des côtés opposés du système de transport.

3. Appareil selon l'une ou l'autre des revendications 1 et 2, dans lequel la source de faisceau électronique (34) comprend des moyens pour générer des impulsions d'électrons ayant une énergie cinétique de 100 à 500 keV, les impulsions durant de 20 à 1 000 nanosecondes.

4. Appareil selon la revendication 3, dans lequel les moyens pour générer les impulsions génèrent des électrons avec une énergie cinétique de 200 keV et les impulsions durent 20 nanosecondes.

5. Appareil selon la revendication 3, dans lequel l'énergie cinétique est de 500 keV et les impulsions durent de 100 à 500 nanosecondes.

6. Appareil selon l'une quelconque des revendications 1 à 5, dans lequel le moyen de transport transporte des billets de banque déposés au niveau du moyen de distribution ou de réception (38) à travers la chambre de traitement vers la boîte de rangement pour des espèces (22).

7. Appareil selon l'une quelconque des revendications 1 à 6, dans lequel le moyen de rayonnement (34) comprend des moyens pour produire un faisceau électronique ayant une coupe transversale comparable en taille à une surface de l'objet plat exposé au rayonnement (12).

8. Procédé de décontamination d'objets minces et plats (12) comprenant des billets de banque contenant potentiellement une contamination microbienne (20), le procédé comprenant :
le passage d'objets plats individuels le long d'un trajet de transport à travers une région de traitement ;
la détection de chaque objet plat entrant dans la région de traitement ;
la génération d'un rayonnement comprenant des impulsions de faisceau électronique allant jusqu'à 500 keV ;
le déclenchement de l'exposition au rayonnement de chaque objet plat avec un sursaut du rayonnement, au fur et à mesure qu'il passe à travers la région de traitement ;
le transport des billets de banque à travers la région de traitement (18) comprise entre une boîte de rangement pour des espèces (22) et un orifice pour billets de banque (14) ; et
le déclenchement de l'étape d'exposition au rayonnement au fur et à mesure que chaque billet de banque se déplace dans la région de traitement.

9. Procédé selon la revendication 8, dans lequel le rayonnement comprend des électrons avec une énergie en dessous de 100 keV.

10. Procédé selon l'une ou l'autre des revendications 8 et 9, dans lequel le rayonnement comprend en outre des rayons X.

11. Procédé selon l'une quelconque des revendications 8 à 10, dans lequel l'étape d'exposition au rayonnement comprend :
l'exposition au rayonnement des objets plats simultanément à partir des côtés opposés.

12. Procédé selon l'une quelconque des revendications 8 à 11, dans lequel l'étape d'exposition au rayonnement comprend :
l'envoi d'impulsions à un faisceau électronique pour générer des impulsions de 20 à 1 000 nanosecondes.

13. Procédé selon l'une quelconque des revendications 8 à 12, dans lequel l'étape d'exposition au rayonnement comprend en outre :
la génération d'impulsions de faisceau électronique d'électrons de 100 à 500 keV.

14. Procédé selon l'une quelconque des revendications 8 à 13, comprenant en outre :
la réception des billets de banque déposés au niveau de l'orifice pour billets de banque ;
le transport des billets de banque déposés à travers la région de traitement vers la boîte de rangement pour des espèces ;
le déclenchement de l'exposition au rayonnement au fur et à mesure que chaque billet de banque déposé se déplace à travers la région de traitement.

15. Procédé selon l'une quelconque des revendications 8 à 14, comprenant en outre l'étape consistant à chauffer la contamination microbienne dans des conditions pratiquement adiabatiques, la chaleur étant créée par l'étape d'exposition au rayonnement.
